# EUROPEAN PATENT APPLICATION

(11) **EP 3 633 686 A1**
(43) Date of publication of application: **08.04.2020**
(21) Application number: 18198590.4
(22) Date of filing: 04.10.2018
(51) Int. Cl.: G21K 1/02, A61B 6/00

(54) **DIRECTIONAL X-RAY BEAM MODIFIER**

(71) Applicant: Koninklijke Philips N.V., 5656 AG Eindhoven (NL)
(72) Inventor: WIMMERS, Onno Jan, 5656 AE Eindhoven (NL); SANDERS, Hubertus Cornelis Gerardus Maria, 5656 AE Eindhoven (NL); PENNERS, Hubertus Johannes Maria, 5656 AE Eindhoven (NL)
(74) Representative: de Haan, Poul Erik

(57) **Abstract**

To produce an X-Ray beam modifier useful in X-Ray imaging system, many thousands of lamellae are fused together in a laborious and fragile process. As improvement, there is provided a directional X-ray beam modifier for insertion into the source to detector axis of an X-ray imager.

The directional X-Ray beam modifier comprises a plurality of X-ray absorbing portions stacked laterally along a direction substantially transverse the source to detector axis in use. A first X-ray absorbing portion of the plurality of X-ray absorbing portions is oriented at a first offset angle from the source to detector axis, and comprises at least two adjacent X-ray absorbing lamellae arranged in parallel to each other and separated from each other by an X-ray transmissive parallel inter-spacer portion.

A second X-ray absorbing portion of the plurality of X-ray absorbing portions is arranged at a second offset angle that is different to the first offset angle.

## Description

### FIELD OF THE INVENTION

This application relates to a directional X-Ray beam modifier, an X-Ray source, a phase-contrast X-Ray imaging system, and method for manufacturing a directional X-ray beam modifier.

### BACKGROUND OF THE INVENTION

X-Ray systems comprise several different components intended to be placed in the X-Ray beam-line to modify the radiation emitted from an X-Ray source in a way that improves the performance of an X-Ray system. For example, an anti-scatter grid comprises a grid with X-Ray absorbing lamellae that diverge outwards slightly between the source and the detector to interrupt scattered X-Ray radiation. A grid filter can be provided to a focussed X-Ray beam. Analyzer gratings (G2 gratings) having a slight divergence for sampling an interference pattern in phase-contrast imaging are another example of such a grid component.

US 9,424,958 discusses a grid filter, an example of an X-Ray beam modifier. Typical grid filters comprise a stack of between 300 and 4,000 individual lamellae, for example. Existing production processes are expensive and often laborious and contribute to the expense of such X-Ray grid filter components.

Accordingly, these components and their production processes may be further improved.

### SUMMARY OF THE INVENTION

Thus, there may be a need to provide an improvement in directional X-Ray beam modifier manufacture.

The object of the present invention is solved by the subject-matter of the independent claims. Further exemplary embodiments are evident from the dependent claims and from the following description.

According to a first aspect, there is provided a directional X-ray beam modifier for insertion into the source to detector axis of an X-ray imager comprising:
- a plurality of X-ray absorbing portions stacked laterally along a direction substantially transverse the source to detector axis in use.

A first X-ray absorbing portion of the plurality of X-ray absorbing portions is oriented at a first offset angle from the source to detector axis, and comprises at least two adjacent X-ray absorbing lamellae arranged in parallel to each other and separated from each other by an X-ray transmissive parallel inter-spacer portion.

A second X-ray absorbing portion of the plurality of X-ray absorbing portions is arranged at a second offset angle that is different to the first offset angle.

Accordingly, by providing a directional X-Ray beam modifier comprising at least two adjacent X-Ray absorbing lamellae arranged in parallel to each other and separated by parallel inter-spacer portion, the assembly of the X-Ray beam modifier from individual X-ray absorbing lamellae separated by X-ray transmissive inter-spacers can be significantly simplified. This is because, for example, in a case where all X-Ray absorbing lamellae are provided in prefabricated pairs, the number of assembly operations is substantially half of the number of assembly operations required otherwise. Furthermore, the X-Ray absorbing lamellae are typically elongated planar metal elements having a thickness of as low as 10 um, and thus the lamellae are fragile and prone to handling damage. Combining two lamellae and a parallel inter-spacer as a unified X-Ray absorbing portion leads to a stiffer element that is more resistant to handling during manufacturing.

Optionally, the second X-ray absorbing portion further comprises at least two further adjacent X-ray absorbing lamellae arranged in parallel to each other and separated from each other by a further X-ray transmissive parallel inter-spacer portion.

Accordingly, a large plurality of X-Ray absorbing portions having parallel lamellae maybe fused together in similar fashion.

Optionally, the directional X-ray beam modifier further comprises:
- at least one X-ray transmissive non-parallel spacer element.

The at least one non-parallel spacer element separates the first X-ray absorbing portion and the second X-ray absorbing portion at the second offset angle.

Accordingly, each X-Ray absorbing portion of the plurality of X-Ray absorbing portions may be offset at different angles to each other (so that the X-Ray absorbing portions are non-parallel to each other) to facilitate manufacturing whilst still enabling acceptable focusing performance.

Optionally, the X-ray absorbing lamellae of the plurality of X-ray absorbing portions are arranged at least pairwise parallel to each other.

Optionally, the plurality of X-ray absorbing portions comprises a stack of between 100 and 10,000 lamellae.

Thus, the technique may be applied to relatively small grids (such as grid filters used in X-Ray sources) or relatively large grids (such as the G2 analyser grating of a phase-contrast X-Ray imaging system).

Optionally, the lamellae of the first X-ray absorbing portion have an average thickness of between 5 um - 75 um.

Optionally, the parallel inter-spacer portion of the first X-ray absorbing portion has an average thickness of between 1 um - 200 um.

Accordingly, the grid assembly technique discussed this application may be applied to a wide range of grid types and applications.

Optionally, a plurality of offset angles relative to the source-detector axis of the plurality of X-ray absorbing portions increases relative to a transverse distance of a respective X-ray absorbing portion of the plurality of X-ray absorbing portions from the source-detector axis in the lateral stack of X-ray absorbing portions to thus form a focus.

Optionally, each lamella out of the plurality of lamellae comprised in the plurality of X-ray absorbing portions has two parallel surfaces elongated in parallel to a radiation propagation direction along the source to detector axis. Each surface of each lamella defines a plane, and the orientation of each plane is defined by a respective surface vector perpendicular to the respective plane. The surface vector of a lamella out of the plurality of lamellae is parallel to the surface vector of a neighboring lamella, and not all of the surface vectors are parallel to each other.

Optionally, the X-ray transmissive parallel inter-spacer portion comprises one of: Aluminum, polycarbonate, epoxy, PMMA, vulcanized fibres, and wherein the X-ray absorbing lamellae comprise Molybdenum, Tin, Silver, Lead, Tungsten, Gold, Platinum, or alloys thereof.

Optionally, the directional X-ray beam modifier according to one of the preceding claims, configured as one of a grid filter for multiple focal spot X-ray imaging, an anti-scatter grid, an analyzer grating for dark-field imaging, or an analyzer grating for phase-contrast imaging.

Accordingly, the grid assembly technique discussed this application may be applied to a wide range of grid types and applications.

According to a second aspect there is provided an X-ray source comprising:
- an anode;
- a cathode, and
- a grid filter and/or an anti-scatter grid in alignment with an X-ray emission direction of the X-ray source, comprising the directional X-ray beam modifier according to the first aspect or its embodiments. The cathode is configured to emit electrons towards the anode to thus cause the emission of X-ray radiation from the anode and through the grid filter and/or the anti-scatter grid.

Accordingly, the cost of assembly of a X-Ray source may be reduced because its grid filter and/or anti-scatter grid can be assembled more efficiently.

According to a third aspect there is provided a phase contrast X-ray imaging system, comprising:
- an X-ray source;
- an X-ray detector;
- a phase grating (G1); and
- an analyzer grating (G2).

The X-ray source is configured to emit X-ray radiation along a source to detector axis towards the X-ray detector through the phase grating and the analyzer grating; and wherein the analyzer grating (G2) and/ or the phase grating (G1) is a directional X-ray beam modifier according to the first aspect or its embodiments.

Accordingly, the cost of assembly of a phase contrast X-ray imaging system may be reduced because its grid filter and/or anti-scatter grid can be assembled more efficiently.

According to a fourth aspect there is provided a method for manufacturing a directional X-ray beam modifier for insertion into the source to detector axis of an X-ray imager comprising:
a) providing a first X-ray absorbing portion of a plurality of X-ray absorbing portions oriented at a first offset angle from a first axis, the first X-ray absorbing portion comprising at least two adjacent X-ray absorbing lamellae arranged in parallel to each other and separated from each other by an X-ray transmissive parallel inter-spacer portion;
b) providing a second X-ray absorbing portion of the plurality of X-ray absorbing portions that is arranged at a second offset angle that is different to the first offset angle from the first axis.

Accordingly, a range of filters for use in a X-ray system, or X-Ray source can be manufactured more efficiently.

Optionally, the method according to the fourth aspect further comprises:
a1) providing a first portion and a second portion of X-ray absorbing lamella blank material
a2) coating a first side of the first portion and/or a first side of the second portion of the X-ray absorbing lamella blank material with a parallel layer of an X-ray transmissive parallel inter-spacer portion material;
b1) coating a first side of the first portion and/or a first side of the second portion of the parallel layer of an X-ray transmissive parallel inter-spacer portion material on the X-ray absorbing lamella or lamellae with glue;
b2) bringing the first side of the first portion and the first side of the second portion of the lamella blank material into a facing spatial relationship; b3) attaching the first side of the first portion and the first side of the second portion of the lamella blank material to form the first X-ray absorbing portion having a parallel inter-spacer portion;
b4) providing a second X-ray absorbing portion having a parallel inter-spacer portion; and
b5) attaching the first X-ray absorbing portion and the second X-ray absorbing portion whilst first and second X-ray absorbing portions are separated by at least one non-parallel spacer element.

In this application, the term "directional X-Ray beam modifier" means a physical element having an X-ray source directable plane, and an X-ray detector directable plane. When placed in a source-detector axis of an X-ray system, with the X-ray source directable plane facing the X-Ray source, and the X-ray detector directable plane facing the X-Ray detector, the directional X-Ray beam modifier changes an output characteristic of an X-Ray beam compared to its input. For example, when used as an anti-scatter grid (a filter placed in-between a patient imaging area and an X-Ray detector, for example) the directional X-Ray beam modifier receives via its source directable plane and X-Ray beam containing a portion of scattered X-Ray radiation, and outputs an X-Ray beam from its X-Ray detector directable plane with substantially all of the scattered X-Ray radiation removed, leaving only the direct X-Ray beam. When used as an analyser grating (G2) in a phase-contrast system, the X-Ray source directable plane faces the phase grating (G1) and spatially samples the Talbot pattern comprising X-Ray phase shift information for a patient, thus emitting a modified X-Ray beam comprising the sampled Talbot pattern towards an X-Ray detector.

In this application, the term "source to detector axis" means an imaginary geometric line in 3D space directed from the point of emission of X-Ray radiation from an X-Ray source to the point at which a X-Ray detector is located (or the average centre of the X-Ray detector in the case of a flat-panel X-Ray detector). A X-Ray fan beam emitted from an X-Ray source generally follows the source detector axis and the fan beam diverges laterally in a plane bisected, for example, by the source to detector axis, although in practical implementations edge effects of the X-Ray beam may also occur.

In this application, the term "an X-Ray absorbing portion" means at least two lamellae (optionally of the same or different lengths, and/or optionally of the same or different thicknesses) that are aligned side-by-side (transversely to each other) and separated by the same distance along the entire length of the lamellae. In other words, the at least two lamellae can be modelled by parallel planes in 3D Euclidean space (imaginary planes that do not intersect or touch each other at any point when extended to infinity in 3D Euclidean space). Accordingly, an X-Ray beam entering the parallel space separating the two lamellae will traverse the parallel space separating the two lamellae if the X-Ray beam is parallel or substantially parallel to the two lamellae. However, if the X-Ray beam entering the parallel space separating the at least two lamellae is not parallel to the at least two lamellae, it will be partially or entirely attenuated by a lamella that it intersects with. Of course, in practice manufacturing tolerancing issues may mean that substantially parallel planes can be provided that are not mathematically parallel, but function in accordance with the description above. Of course, an X-Ray absorbing portion may comprise at least three parallel lamella, at least four parallel lamellae, at least five parallel lamellae, or more.

In this application, the term "first offset angle" is the acute angle (more than 0 degrees but less than 90 degrees) enclosed by an imaginary line parallel to and mid-way in-between at least the first and second lamellae of the X-Ray absorbing portion and intersecting with the imaginary line (source-detector axis) between the source and detector. A plurality of X-Ray absorbing portions will comprise a series of offset angles incrementing and decrementing in series relative to the source-detector axis. An X-Ray absorbing portion bisected between the first and second lamellae by the source-detector axis has a first offset angle of zero degrees.

Furthermore, because the first and second lamellae of an X-Ray absorbing portion are parallel and separated by a non-trivial distance, it is not possible for both of the first and second lamellae to be parallel to an imaginary plane in between the source and the respective first and second lamellae. The X-Ray absorbing portion may be angled so that its first lamella is parallel with an imaginary claim in between the source and the first lamella. Alternatively, the X-Ray absorbing portion may be angled so that its second lamella is parallel with an imaginary plane in-between the second lamella and the source. Alternatively, the X-Ray absorbing portion may be angled at a range of intermediate angles representing a compromise between either the first or the second lamella being oriented parallel to respective planes between the first or the second lamellae and the source. Accordingly, it will be appreciated that the optical efficiency (the amount of X-Ray radiation conducted from the source to the detector) of the X-Ray absorbing portion is slightly reduced compared with a case where every lamella is oriented at a slightly different angle. However this slight reduction in optical efficiency is tolerable in view of the great manufacturing efficiencies possible.

In the following application, the term "X-Ray absorbing" is a quality in particular of the lamellae of the X-Ray absorbing portion having a mass attenuation coefficient similar to that of Molybdenum, Tungsten, Lead, and the like.

In the following application, the term "X-Ray transmissive" is a quality in particular of the material in the interspacer portion between parallel lamellae and may have a mass attenuation coefficient similar to that of air, plastic, polymer, epoxy resin, vulcanized fibre, and the like.

In the following application, the term "non-parallel spacer element" refers to an element provided in the space between two adjacent X-Ray absorbing portions. At least two lamellae of an X-Ray absorbing portion are parallel to each other. However, the X-Ray absorbing portions themselves are non-parallel to each other. Accordingly the spacer element separating a first and a second X-Ray absorbing portion has a first surface facing a first X-Ray absorbing portion and a second surface facing a second X-Ray absorbing portion. The planes of the first and second surfaces are non-parallel in 3D Euclidean space. For example, the non-parallel spacer elements may be a triangle, or a non-square, non-rectangular quadrilateral. In other words, the non-parallel spacer element generates an offset angle increment between a first X-Ray absorbing portion and a second X-Ray absorbing portion relative to a source-detector axis.

In the following application, the term "at least pairwise parallel" means that, for plurality of lamellae, the plurality is divided into disjoint groups. The planes of the lamellae of the same group are parallel to each other in 3D Euclidean space. The planes of the lamellae of different groups are non-parallel to each other in 3D Euclidean space (a group is equivalent to an X-Ray absorbing portion). Although the preferred number of lamellae per group is two, each disjoint group may comprise three, four, five, six, or more lamellae.

Accordingly, a basic idea of the invention is that multiple focal spot spectral X-Ray imaging, for example, requires a grid used as a spectral filter having thinner X-Ray absorbing lamellae and reduced interspaces compared to conventional X-Ray anti-scatter grids. As a consequence, during manufacturing, when assembling a grid filter from individual lamellae the individual lamellae are relatively thin. This results in increased pliability during handling and assembly. To overcome this, it is proposed firstly to attach two pieces of individual lamellae material and spacer material together to obtain a thicker composite material before assembling. The strips have increased stiffness before assembling, allowing for easier handling. After assembling, the grid filter (for example) will consist of at least parallel pairs (possibly triplets or quadruplets or more) of individual lamellae arranged in a focus pattern. Although there is a slight degradation in optical performance, the arrangement still works acceptably as (for example) a grid filter and leads to significant manufacturing efficiencies.

### BRIEF DESCRIPTION OF THE DRAWINGS

Fig 1a) schematically illustrates a side cut-through view of a directional X-Ray beam modifier in accordance with the first aspect.
Fig 1b) schematically illustrates a side cut-through view of a pair of lamellae and a parallel portion of inter-spacer material forming an X-Ray absorbing portion.
Fig 1c) schematically illustrates another side cut-through view of a directional X-Ray beam modifier in accordance with the first aspect.
Fig 2 schematically illustrates a side cut-through view of a subset of lamellae of a directional X-Ray beam modifier in accordance with the first aspect in relation to a source location.
Fig 3 schematically illustrates a side cut-through view of an X-ray source in accordance with the second aspect.
Fig 4 schematically illustrates a phase contrast X-ray imaging system in accordance with the third aspect.
Fig 5 schematically illustrates a method according to a fourth aspect.

### DETAILED DESCRIPTION OF EMBODIMENTS

A conventional grid filter for multiple focal spot X-Ray radiation filtering to achieve spectral X-Ray imaging is discussed, for example, in US 9,424,958. Such a grid filter comprises a stack of focused lamellae of material (such as Mo, Sn, Ag, for example) that filters incoming X-rays. A low X-Ray absorptive material (such as polycarbonate or epoxy) is used as the inter-spacer between the focused lamellae. Such a grid filter may typically have a height of between 1 mm and 10 mm and comprise a stack of between 300 and 4,000 individual lamellae. In the conventional X-Ray grid filter, each lamella of the grid filter is substantially coplanar to a respective plane in 3D Euclidean space intersecting the source location. In other words, in the conventional X-Ray grid filter, none of the lamellae are in a plane that is parallel to the plane of another lamella of the conventional grid filter. Although this provides theoretically approaches optimal optical (focal) efficiency, such a filter is difficult to manufacture because every lamella must be individually spaced from its neighboring lamella.

A typical manufacturing process is (i) to coat the lamella material with inter-spacer material (such as epoxy), (ii) to assemble individual strips of coated lamella material to form a focused grid filter.

However, the lamella thickness is typically between 10 and 30 µm. Handling the individual strips is difficult without causing damage to them. Furthermore, a typical grid filter may contain up to 4000 individual lamellae, each requiring careful handling.

As a solution, it is proposed to provide according to a first aspect of the invention a directional X-ray beam modifier 10 for insertion into the source to detector axis SD of an X-ray imager comprising a plurality of X-ray absorbing portions P₁, P₂, P_{N} stacked laterally along a direction D_{T} substantially transverse the source to detector axis SD in use.

A first X-ray absorbing portion P₁ of the plurality of X-ray absorbing portions is oriented at a first offset angle θ*ₒ* from the source to detector axis, and comprises at least two adjacent X-ray absorbing lamellae *L*₁, *L*₂ arranged in parallel to each other and separated from each other by an X-ray transmissive parallel inter-spacer portion 12.

A second X-ray absorbing portion P_{N} of the plurality of X-ray absorbing portions is arranged at a second offset angle *θ_{N}* that is different to the first offset angle. Optionally the second X-ray absorbing portion is immediately adjacent to the first X-ray absorbing portion P₁.

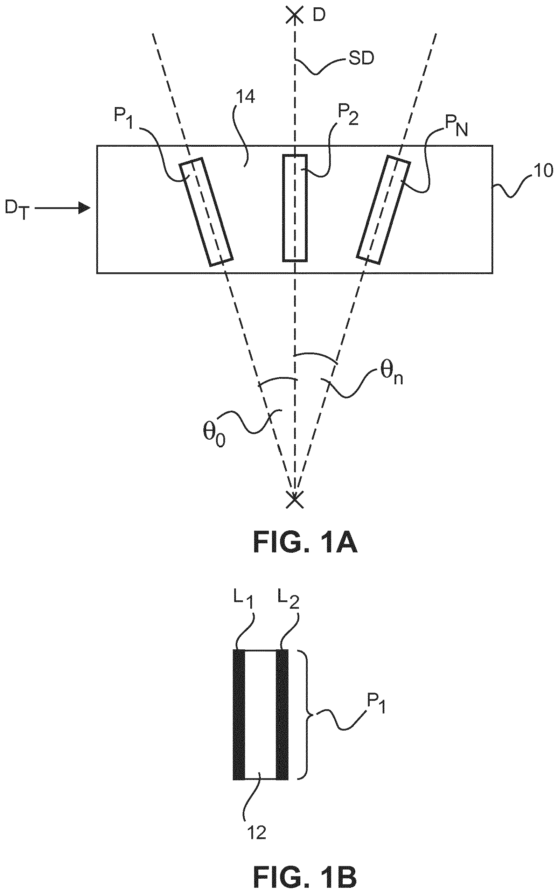

Fig 1a) schematically illustrates a side cut-through view of a directional X-Ray beam modifier 10 in accordance with the first aspect. This drawing is a simplified block-diagram representation. The X-Ray absorbing portions P₁ - P_{N} are represented in figure 1a) as rectangles. However, a subset P₁ of the total number of absorbing portions P_{N} are implemented as shown in Fig 1b). The X-Ray absorbing portion P₁ is aligned at a first offset angle θ*ₒ* from the source to detector axis SD. The X-Ray absorbing portion P_{N} is aligned at a second offset angle *θ_{N}* from the source to detector axis SD.
A special case in Fig 1a) is X-Ray absorbing portion P₂ because it is substantially bisected by the source to detector axis SD. Therefore, X-Ray absorbing portion P₂ will have an offset angle of substantially 0 degrees.

Fig 1b) schematically illustrates a side cut-through view of a pair of lamellae and a parallel portion of inter-spacer material forming an X-Ray absorbing portion. Lamellae L₁, L₂ are comprised of, for example, a thin planar portion of Mo, Sn or Ag. In Fig 1b), the lamellae are displayed with their thinnest surfaces facing out of and into the page. The lamellae L₁, L₂ may both have an average thickness in the range 1 to 100 um. The lamellae L₁, L₂ may both have an average thickness in the range 10 to 80 um, 30 to 70 um, 40 to 60um, or 45 to 55 um. Optionally, a first lamella of the X-Ray absorbing portion L₁ has a different thickness to a second lamella L₂ of the X-Ray absorbing portion. Optionally, the first lamella L₁ of the X-Ray absorbing portion P₁ comprises a different material to the second lamella L₂ of the X-Ray absorbing portion.

Lamellae L₁ and L₂ are separated by a X-ray transmissive parallel inter-spacer portion 12. Accordingly, the plane defined in 3D Euclidean space by lamella L₁ is parallel to the plane defined in 3D Euclidean space by lamella L₂. The X-ray transmissive parallel inter-spacer portion 12 is parallel to both the lamella L₁ and L₂. The X-ray transmissive parallel inter-spacer portion 12 a provided, for example, by a parallel portion of epoxy, although other materials and even low Z metals (such as aluminum) can also form the inter-spatial portion. The inter-spacer portion may have a thickness of between 20 and 100 µm. Optionally, the inter-spacer portion has a thickness of above 20 um, above 30 um, above 40 um, above 50 um, above 60 um, above 100 um, or above 150 um.

It will be appreciated that because one or both lamellae are coated with a thin layer of glue, the inter-spacer portion can comprise a laminar stack of a first glue portion, the interspacer material, and a second glue portion without loss of generality, because glues typically have a mass absorption coefficient similar to the inter-spacer material and different to the lamella material. Optionally, the parallel inter-spacer position entirely comprises glue. Accordingly, the X-Ray absorbing portion P₁ maybe mass produced separately (at a different time, in a different place, a different part of the manufacturing process) to the entire directional X-ray beam modifier 10. Furthermore, because the X-Ray absorbing portion P₁ comprises at least two lamellae L₁ and L₂ supported on a parallel inter-spacer portion 12, the fragile material of the lamellae L₁ and L₂ is strengthened and more resistant to production stress.

Fig 1a) also schematically illustrates at least one X-ray transmissive non-parallel spacer element 14. The at least one non-parallel spacer element 14 separates the first X-ray absorbing portion P₁ and the second X-ray absorbing portion P₂ at the second offset angle. In other words, the side-section of the non-parallel spacer element 14 is, for example, in section a quadrilateral having sides that face adjacent X-Ray absorbing portions P₁ and P₂. However, because the adjacent X-Ray absorbing portions P₁ and P₂ are each angled towards the X-Ray source S, their opposing sides are not in parallel planes.

Optionally, the X-ray transmissive non-parallel spacer element 14 comprises the same material as used for the parallel inter-spacer portions 12. Optionally, the X-ray transmissive non-parallel spacer element 14 is a different material compared used for the parallel inter-spacer portions 12. The incremental effect of the sides of the multiple non-parallel spacer elements 14 is to produce a stack of focused X-Ray absorbing portions P₁ to P_{N}. Optionally, at least one X-Ray absorbing portion P₁ may be provided with the non-parallel spacer element 14 pre-attached to one side of the X-Ray absorbing portion in the transverse direction.

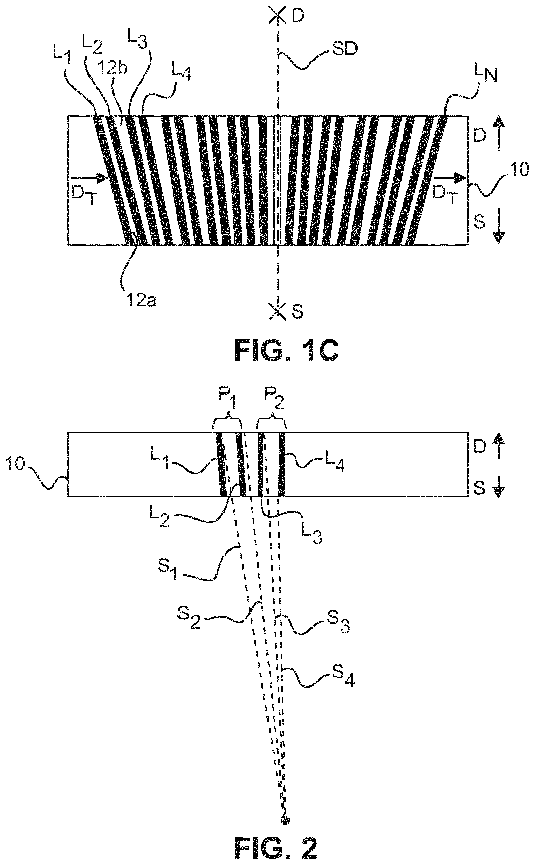

Fig 1c) schematically illustrates another side cut-through view of a directional X-Ray beam modifier 10 in accordance with the first aspect. In Fig 1c), the directional X-Ray beam modifier 10 is illustrated showing a plurality of lamellae L₁...L_{N} directly, rather than the boxes representing the X-Ray absorbing portions P₁ ... P_{N} of Fig 1a). In Fig 1c), lamellae L₁ and L₂ are parallel to each other and together with their interspace material 12 form a first X-Ray absorbing portion. Neither of lamellae L₁ and L₂ or their interspace material 12 are in parallel to neighboring lamella L₃. In other words, in the illustrated X-Ray beam modifier 10 of figure Fig 1c) the group of lamellae L₁ and L₂ form a parallel pair. The group of lamellae L₃ and L₄ form an adjacent parallel pair. Lamellae L₁-L₄ are thus disjoint pairwise parallel. In other words, the group of Lamellae L₁-L₄ comprises a first disjoint pair of parallel lamellae L₁ and L₂, and an immediately neighboring (adjacent) disjoint pair of parallel lamellae L₃ and L₄. In other words, a lamella selected from the first (L₁, 12a, L₂) absorbing portion is non-parallel to any lamella selected from the second (L₃, 12b, L₄) X-Ray absorbing portion.

X-Ray absorbing portions P₁ ... P_{N} are stacked transversely in focus to form the final directional X-Ray beam modifier 10.

The X-Ray beam modifier 10 illustrated in Fig 1c) is directional (in the words, only functions when an X-Ray source S is positioned on the "S" side of the X-Ray beam modifier 10 and an X-Ray detector D is positioned on the "D" side of the X-Ray beam modifier 10. This is a result of the divergence direction of the lamellae of the X-Ray beam modifier as known to a person skilled in the art.

Fig 2 schematically illustrates a side cut-through view of a subset of lamellae of a directional X-Ray beam modifier 10 in accordance with the first aspect in relation to a source location S.

In particular, Fig 2 shows a first X-Ray absorbing portion P₁ and a second X-Ray absorbing portion X-Ray absorbing portion P₂. First X-Ray absorbing portion P₁ comprises a first and a second parallel lamella. This illustrates a focal spot position S in which the minimum absorbance by the lamellae occurs. Second X-Ray absorbing portion P₂ comprises a third and a fourth parallel lamella. The first and the second lamellae are not parallel to the third and fourth lamella. Ideal beam lines S₁...S₄ illustrates a straight-line path of an X-Ray beam projected between a single X-Ray source location S and the X-Ray detector directed face of the directional X-Ray beam modifier 10. Accordingly, neither lamella L₁ nor lamella L₂ are perfectly aligned with their respective ideal matching beamlines S₁ and S₂. However, it can be seen from Fig 2 that the angular error arising as a consequence of grouping lamellae L₁ and L₂ in parallel is somewhat minimal.

Because the focus of the directional X-Ray beam modifier 10 is made of pairs of parallel lamellae in the first aspect, the focusing performance is slightly sub-optimal. Compared to the ideal situation in which all single lamellae are focused, a higher fraction of the X-rays incident on the directional X-Ray beam modifier 10 will intersect with the X-Ray absorbing lamellae and pass through less X-Ray absorbing material. However, for grid filter dimensions applicable to a typical X-Ray grid filter (approximately 10 mm x 10 mm)this does not prevent meaningful spectral separation of the X-rays at the two focal spot positions. Grids forming an X-Ray anti-scatter filter (approximately 400mm x 400mm) and phase-contrast X-Ray absorption gratings G2 (approximately 400mm x 400mm) constructed according to the first aspect also do not suffer a significant decline in the resolution of their output images.

In geometric terms each lamella out of a plurality of lamellae comprised in the plurality of X-ray absorbing portions has two parallel surfaces elongated in parallel to a radiation propagation direction along the source to detector axis. Each surface of each lamella defines a plane, and the orientation of each plane is defined by a respective surface vector perpendicular to the respective plane. The surface vector of a lamella out of the plurality of lamellae is parallel to the surface vector of a neighboring lamella not all of the surface vectors are parallel to each other.

As discussed in the summary of invention, the manufactured directional X-Ray beam modifier 10 may be applied in at least three different parts of the typical X-Ray beam line in typical medical X-Ray image scanners. Table 1 illustrates typical ranges of average thickness of the inter-spacer material and X-Ray absorbing material in each application.

**Table 1**

| | ***X-ray absorbing material*** | | ***Inter spacer material*** | |
|---|---|---|---|---|
| | ***Average Thickness*** | | ***Average Thickness*** | |
| ***Application*** | ***Min*** | ***Max*** | ***Min*** | ***Max*** |
| Anti-Scatter Grid | 10 µm | 75 µm | 50 µm | 300 µm |
| Dark Field G2 grating | 5 µm | 30 µm | 5 µm | 50 µm |
| Grid Filter | 5 µm | 75 µm | 5 µm | 300 µm |

According to a second aspect, there is provided an X-ray source 20. The X-Ray source 20 comprises:
- an anode 22;
- a cathode 24, and
- a grid filter 26 and/or an anti-scatter grid in alignment with an X-ray emission direction of the X-ray source, comprising the directional X-ray beam modifier according to the first aspect or one of its embodiments. The cathode is configured to emit electrons towards the anode to thus cause the emission of X-ray radiation 28 from the anode and through the grid filter and/or the anti-scatter grid.

Optionally, a directional X-ray beam modifier 10 is provided according to the first aspect in which a first region of the directional X-ray beam modifier 10 comprises a sub-stack of pairwise parallel lamellae, and a second, adjacent, region of the directional X-Ray beam modifier 10 comprises a further sub-stack of non-parallel lamellae. Optionally, the non-parallel lamellae are disposed in the centre of the directional X-ray beam modifier 10 (closest to the focus). Optionally, a first sub-stack of pairwise parallel lamellae is provided adjacent to a first edge of the directional X-ray beam modifier 10, and the second sub-stack of pairwise parallel lamellae is provided adjacent to a second edge of the directional X-Ray beam modifier 10. Optionally, the first and second sub-stacks occupy total proportion of 5%, 10%, 15%, 25%, 50%, 75%, or 90% of the total stack of lamellae.

According to the foregoing embodiment, a central area (near focus) of the directional X-Ray beam modifier requiring optimal or better focal performance than peripheral areas of the remainder of the directional X-Ray beam modifier is manufactured with a stack of non-parallel lamellae according to the conventional method. However this central area is surrounded on one or both sides by sub-stacks of pairwise parallel lamellae according to the aspects discussed herein. Accordingly, at regions of the directional X-Ray beam modifier 10 where resolution is important, the non-parallel lamellae provide improved resolution. Conversely, at extreme lateral regions of the directional X-Ray be modifier 10 where resolution may be less important, the new technique outlined herein is employed so that a manufacturing benefit can be gained.

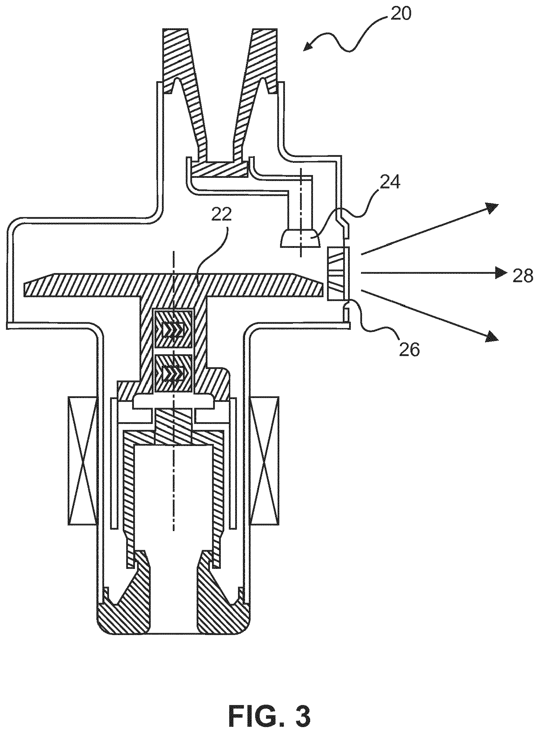

Fig 3 schematically illustrates a side cut-through view of a rotating anode X-ray source in accordance with the second aspect, although be appreciated that other types of X-Ray source can be combined with the directional X-ray beam modifier 10 according to the first aspect.

According to a third aspect, there is provided a phase contrast X-ray imaging system 30, comprising:
- an X-ray source 32;
- an X-ray detector 34;
- a phase grating G1; and
- an analyzer grating G2;
wherein the X-ray source is configured to emit X-ray radiation along a source to detector axis towards the X-ray detector through the phase grating and the analyzer grating; and wherein the analyzer grating G2 is a directional X-ray beam modifier 10 according to the first aspect or one of its embodiments. Optionally, the X-Ray source 32 may also comprise a grid filter (not shown) according to the first aspect or its embodiments.

Turning now to a method of manufacture of the directional X-ray beam modifier 10, as explained above the number of individual lamella manipulation operations can be reduced by at least half when all X-Ray absorbing portions of the directional X-Ray beam modifier 10 are provided according to the present method, resulting in a substantial time saving (owing to fewer lamella manipulation steps). Furthermore, parallel lamellae fused by their parallel interspace portions are significantly less fragile and susceptible to damage during manufacture.

According to fourth aspect, there is provided a method for manufacturing a directional X-ray beam modifier for insertion into the source to detector axis of an X-ray imager. The method comprises:
a) providing a first X-ray absorbing portion of a plurality of X-ray absorbing portions oriented at a first offset angle from a first axis, the first X-ray absorbing portion comprising at least two adjacent X-ray absorbing lamellae arranged in parallel to each other and separated from each other by an X-ray transmissive parallel inter-spacer portion;
b) providing a second X-ray absorbing portion of the plurality of X-ray absorbing portions that is arranged at a second offset angle that is different to the first offset angle from the first axis.

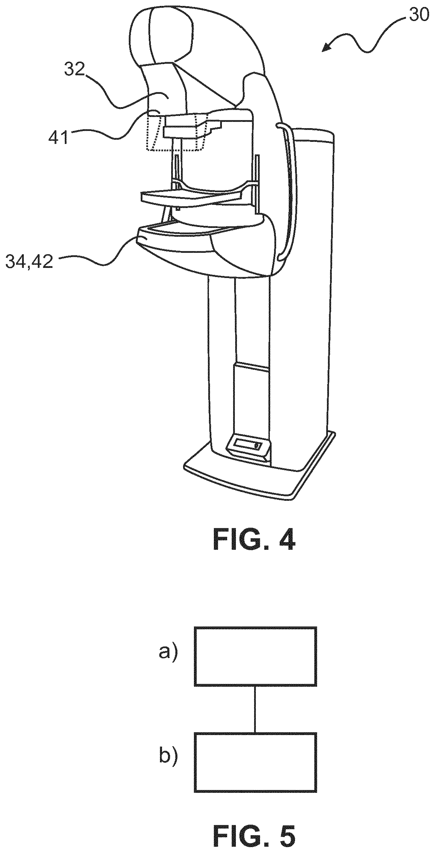

Fig 5 illustrates a method in accordance with a fourth aspect.

Optionally, the method further comprises:
a1) providing a first portion and a second portion of X-ray absorbing lamella blank material
a2) coating a first side of the first portion and/or a first side of the second portion of the X-ray absorbing lamella blank material with a parallel layer of an X-ray transmissive parallel inter-spacer portion material;
b1) coating a first side of the first portion and/or a first side of the second portion of the parallel layer of an X-ray transmissive parallel inter-spacer portion material on the X-ray absorbing lamella or lamellae with glue;
b2) bringing the first side of the first portion and the first side of the second portion of the lamella blank material into a facing spatial relationship; b3) attaching the first side of the first portion and the first side of the second portion of the lamella blank material to form the first X-ray absorbing portion having a parallel inter-spacer portion;
b4) providing a second X-ray absorbing portion having a parallel inter-spacer portion; and
b5) attaching the first X-ray absorbing portion and the second X-ray absorbing portion whilst first and second X-ray absorbing portions are separated by at least one non-parallel spacer element.

For example, two pieces of lamella material can be coated with inter-spacer material. Then, both pieces of coated lamella material may be glued together to form a composite strip. Subsequently, the coated and glued composite strips may be cut into individual composite strips sized to the particular end product. Because the individual composite comprise at least two lamellae and their interspacer glue, they are significantly more robust than individual lamellae. Furthermore, handling steps of fragile individual lamellae are reduced in the manufacturing process. Finally, the composite strips are stacked to form a directional X-Ray beam modifier (such as a grid filter, an anti-scatter grid, or an absorption grating). The non-parallel inter-spacer portions (also of glue, or epoxy, for example) are provided successively as the composite strips are built up into the end product having a focused pattern.

It should be noted that aspects and embodiments of the invention have been described with reference to different subject matter. In particular, some embodiments are described with reference to method-type claims, whereas other embodiments are described with reference to device-type claims. A person skilled in the art will, however, gather from the above and following description, that, unless otherwise notified, in addition to any combination of features belonging to one type of subject-matter, also any combination between features related to different subject-matter is considered to be disclosed with this application. All features discussed herein can be combined providing synergetic effects that are more than simply a summation of the features. While the invention has been illustrated and described in detail in the drawings and foregoing description, such illustration and description are to be considered illustrative or exemplary, and not restrictive. The invention is not limited to the disclosed embodiments. Other variations to the disclosed embodiments can be understood and effected by those skilled in the art practising the claimed invention from a study of the drawings, the disclosure, and the dependent claims.

In the claims, the word "comprising" does not exclude other elements or steps and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A directional X-ray beam modifier (10) for insertion into the source to detector axis (SD) of an X-ray imager comprising:
- a plurality of X-ray absorbing portions (P₁...P_{N}) stacked laterally along a direction substantially transverse the source to detector axis in use;
wherein a first X-ray absorbing portion (P₁) of the plurality of X-ray absorbing portions is oriented at a first offset angle (θ_{ο}) from the source to detector axis, and comprises at least two adjacent X-ray absorbing lamellae (L₁, L₂) arranged in parallel to each other and separated from each other by an X-ray transmissive parallel inter-spacer portion (12); and
wherein a second X-ray absorbing portion (P₂) of the plurality of X-ray absorbing portions is arranged at a second offset angle that is different to the first offset angle.

2. The directional X-ray beam modifier (10) according to claim 1,
wherein the second X-ray absorbing portion (P₂) further comprises at least two further adjacent X-ray absorbing lamellae (L₃, L₄) arranged in parallel to each other and separated from each other by a further X-ray transmissive parallel inter-spacer portion.

3. The directional X-ray beam modifier (10) according to one of claims 1 or 2, further comprising:
- at least one X-ray transmissive non-parallel spacer element (14);
wherein the at least one non-parallel spacer element separates the first X-ray absorbing portion (P₁) and the second X-ray absorbing portion (P₂) at the second offset angle.

4. The directional X-ray beam modifier (10) according to one of the preceding claims,
wherein the X-ray absorbing lamellae of the plurality of X-ray absorbing portions (P₁...P_{N}) are arranged at least pairwise parallel to each other.

5. The directional X-ray beam modifier (10) according to one of the preceding claims,
wherein the plurality of X-ray absorbing portions (P₁...P_{N}) comprises a stack of between 100 and 10,000 lamellae.

6. The directional X-ray beam modifier (10) according to one of the preceding claims,
wherein the lamellae (L₁, L₂) of the first X-ray absorbing portion (P₁) have an average thickness of between 5 um - 75 um.

7. The directional X-ray beam modifier (10) according to one of the preceding claims,
wherein the parallel inter-spacer portion (12) of the first X-ray absorbing portion (P₁) has an average thickness of between 1 um - 200 um.

8. The directional X-ray beam modifier (10) according to one of the preceding claims,
wherein a plurality of offset angles relative to the source-detector axis of the plurality of X-ray absorbing portions (P₁...P_{N}) increases relative to a transverse distance of a respective X-ray absorbing portion of the plurality of X-ray absorbing portions (P₁...P_{N}) from the source-detector axis in the lateral stack of X-ray absorbing portions to thus form a focus.

9. The directional X-ray beam modifier (10) according to one of the preceding claims,
wherein each lamella out of a plurality of lamellae comprised in the plurality of X-ray absorbing portions has two parallel surfaces elongated in parallel to a radiation propagation direction along the source to detector axis;
wherein each surface of each lamella defines a plane;
wherein the orientation of each plane is defined by a respective surface vector perpendicular to the respective plane;
wherein the surface vector of a lamella out of the plurality of lamellae is parallel to the surface vector of a neighboring lamella; and
wherein not all of the surface vectors are parallel to each other.

10. The directional X-ray beam modifier (10) according to one of the preceding claims,
wherein the X-ray transmissive parallel inter-spacer portion comprises one of:
Aluminum, polycarbonate, epoxy, PMMA, vulcanized fibre, and wherein the X-ray absorbing lamellae comprise Molybdenum, Tin, Silver, Lead, Tungsten, Gold, Platinum, or alloys thereof.

11. The directional X-ray beam modifier (10) according to one of the preceding claims, configured as a grid filter for multiple focal spot X-ray imaging, an anti-scatter grid, an analyzer grating for dark-field imaging, or an analyzer grating for phase-contrast imaging.

12. An X-ray source (20) comprising:
- an anode (22);
- a cathode (24), and
- a grid filter (26) and/or an anti-scatter grid in alignment with an X-ray emission direction of the X-ray source, comprising the directional X-ray beam modifier according to one of claims 1-11;
wherein the cathode is configured to emit electrons towards the anode to thus cause the emission of X-ray radiation from the anode and through the grid filter and/or the anti-scatter grid.

13. A phase contrast X-ray imaging system (30), comprising:
- an X-ray source (32);
- an X-ray detector (34);
- a phase grating (G1); and
- an analyzer grating (G2);
wherein the X-ray source is configured to emit X-ray radiation along a source to detector axis towards the X-ray detector through the phase grating and the analyzer grating; and wherein the analyzer grating (G2) is a directional X-ray beam modifier (10) according to one of claims 1-11.

14. A method for manufacturing a directional X-ray beam modifier for insertion into the source to detector axis of an X-ray imager comprising:
a) providing a first X-ray absorbing portion of a plurality of X-ray absorbing portions oriented at a first offset angle from a first axis, the first X-ray absorbing portion comprising at least two adjacent X-ray absorbing lamellae arranged in parallel to each other and separated from each other by an X-ray transmissive parallel inter-spacer portion;
b) providing a second X-ray absorbing portion of the plurality of X-ray absorbing portions that is arranged at a second offset angle that is different to the first offset angle from the first axis

15. The method according to claim 14, further comprising:
a1) providing a first portion and a second portion of X-ray absorbing lamella blank material
a2) coating a first side of the first portion and/or a first side of the second portion of the X-ray absorbing lamella blank material with a parallel layer of an X-ray transmissive parallel inter-spacer portion material;
b1) coating a first side of the first portion and/or a first side of the second portion of the parallel layer of an X-ray transmissive parallel inter-spacer portion material on the X-ray absorbing lamella or lamellae with glue;
b2) bringing the first side of the first portion and the first side of the second portion of the lamella blank material into a facing spatial relationship;
b3) attaching the first side of the first portion and the first side of the second portion of the lamella blank material to form the first X-ray absorbing portion having a parallel inter-spacer portion;
b4) providing a second X-ray absorbing portion having a parallel inter-spacer portion; and
b5) attaching the first X-ray absorbing portion and the second X-ray absorbing portion whilst first and second X-ray absorbing portions are separated by at least one non-parallel spacer element.
